# EUROPÄISCHE PATENTSCHRIFT

(11) **EP 4 362 999 B1**
(45) Veröffentlichungstag und Bekanntmachung des Hinweises auf die Patenterteilung: **18.06.2025**
(21) Anmeldenummer: 22741202.0
(22) Anmeldetag: 29.06.2022
(51) Int. Cl.: A61M 1/36, A61M 60/109, A61M 60/38, A61M 60/90

(54) **EXTRAKORPORALE KREISLAUFUNTERSTÜTZUNG**
EXTRACORPOREAL CIRCUIT SUPPORT
SUPPORT DE CIRCUIT EXTRACORPOREL

(30) Priorität: 29.06.2021 DE 102021116747
(43) Veröffentlichungstag der Anmeldung: 08.05.2024
(73) Patentinhaber: Hübler, Michael, 8634 Hornbrechtikon (CH); Eidgenössische Technische Hochschule Zürich, 8092 Zürich (CH)
(72) Erfinder: SCHMID, Marianne, 8200 Schaffhausen (CH); MEBOLDT, Mirko, 78462 Konstanz (DE); SOLLBERGER, Samuel, 8057 Zürich (CH); SCHMIADY, Martin Oliver, 8800 Thalwil (CH); HOFMANN, Michael, 8126 Zumikon (CH)
(74) Vertreter: Mertzlufft-Paufler, Cornelius
(86) Internationale Anmeldenummer: PCT/EP2022/067958
(87) Internationale Veröffentlichungsnummer: WO 2023/275174

(56) Entgegenhaltungen:
- WO-A1-2021/056091
- US-A1- 2015 025 448
- US-B1- 6 929 777

## Beschreibung

Die Erfindung betrifft eine extrakorporale mechanische Kreislaufunterstützung mit Herz-Kreislauf und Lungenfunktion mit einer Flüssigkeits-Hauptpumpe, deren Pumpeneinlass über wenigstens eine erste Flüssigkeitsleitung und deren Pumpenauslass über mindestens eine zweite Flüssigkeitsleitung an den Blutkreislauf eines Patienten anschließbar ist, mit einem Oxygenator zum Anreichern des in der mindestens einen zweiten Flüssigkeitsleitung zum Patienten geführten Blutes mit Sauerstoff, sowie mit einem Pumpenantrieb, der die Flüssigkeits-Hauptpumpe antreibt.

Die extrakorporale mechanische Kreislaufunterstützung kann auch als Vorrichtung zur extrakorporalen mechanischen Kreislaufunterstützung bezeichnet werden.

US 2015 025 448 A1 offenbart eine Anordnung mit einer Blutpumpe und einem Gasaustauscher zur extrakorporalen Membranoxygenierung, wobei die Blutpumpe als pulsierende Blutpumpe ausgebildet ist und mit dem Gasaustauscher im gleichen Gehäuse angeordnet ist, und wobei die pulsatile Blutpumpe und der Gasaustauscher mit der gleichen Gasquelle in Verbindung stehen.

US 6 292 777 B1 offenbart eine Flüssigkeitspumpe, die zur Zirkulation und Sauerstoffanreicherung von Blut verwendet wird, wobei die Flüssigkeitspumpe einen elektrischen oder pneumatischen Motor mit einer Motorwelle, eine Gasaustauschanordnung, die mit einem Ende der Motorwelle verbunden ist, und ein hohles Venturielement aufweist, das eine Venturi-Einlassöffnung, eine Venturi-Auslassöffnung und eine Venturi-Ansaugöffnung definiert, wobei die Venturi-Ansaugöffnung in Fluidverbindung mit der Gasaustauschanordnung steht, um ein kohlendioxidreiches Gas anzusaugen.

WO 2021 056 091 A1 offenbart eine Vorrichtung und Verfahren zur Blutfiltration mit semipermeabler Membran, die ein kontrolliertes Filtrat-zu-Nicht-Filtrat-Trennungsverhältnis, eine selbstregulierende Druckverstärkung und eine Energierückgewinnungseinrichtung aufweist und bei der grundlegende Ausführungsformen ohne die Notwendigkeit von Ventilen oder anderen Steuereinrichtungen funktionieren können. Diese Fähigkeiten werden durch ein auf einer Verdrängerpumpe und einem Motor basierendes Gerät bereitgestellt, bei dem die größere volumetrische Verdrängung einer stromaufwärts gelegenen Hydraulikpumpe nicht vollständig von einem stromabwärts gelegenen Hydraulikmotor mit geringerer volumetrischer Verdrängung aufgenommen werden kann, da sie synchron im selben Fluidkreislauf arbeiten. Dies führt zu einer Druckverstärkung in dem Teil des Flüssigkeitskreislaufs, der sich zwischen der Pumpe und dem Motor befindet, so dass ein Flüssigkeitsvolumen, das im Wesentlichen der volumetrischen Verdrängungsdifferenz zwischen der Pumpe und dem Motor entspricht, gezwungen wird, aus dem Kreislauf zu fließen, indem es durch die Poren einer halbdurchlässigen Membran (oder mehrerer), die sich darin befindet, hindurchgeht.

Die Erfindung betrifft weiter eine MRT-Anordnung mit einem Magnetresonanztomographen (MRT) und einer extrakorporalen Kreislaufunterstützung.

Technisch wird der extrakorporale Herz-Lungen-Kreislauf häufig mit Herz-Lungen-Maschinen umgesetzt. Sie werden benötigt, um die Pumpfunktion des Herzens und die Lungenfunktionen hinsichtlich Sauerstoffanreicherung (Oxygenierung) des Blutes und hinsichtlich der Kohlendioxid-Elimination für einen begrenzten Zeitraum zu ersetzen, um dadurch beispielsweise eine Operation am offenen Herzen zu ermöglichen. Dabei wird das Blut über einen kardiopulmonalen Bypass der extrakorporalen Herz-Lungen-Maschine zugeführt, darin mit Sauerstoff angereichert und von Kohlendioxid befreit und anschließend in einem Kreislauf dem Körper des Patienten wieder zugeführt. Für den Zeitraum der offenen Herzoperation kann nämlich das Herz seine Grundfunktion der Blutbeförderung nicht wahrnehmen; damit dennoch alle Organe mit Blut und somit mit Sauerstoff versorgt werden können, wird die Herz-Lungen-Maschine eingesetzt. Diese Herz-Lungen-Maschine besteht hauptsächlich aus einer auch als künstliches Herz dienenden Flüssigkeits-Hauptpumpe (= Blutpumpe) und einer künstlichen Lunge (= Oxygenator), wobei ein Flusssensor den Blutfluss und ein Drucksensor den Blutdruck messen kann.

Als Flüssigkeits-Haupt- oder Blutpumpen werden zurzeit im Wesentlichen zwei Pumpenarten eingesetzt, nämlich
- Kreiselpumpen, auch Zentrifugalpumpen genannt oder Axialpumpen, sowie
- Peristaltikpumpen, auch Rollerpumpen oder Schlauchpumpen genannt.

Herz-Lungen-Maschinen werden im Wesentlichen nur während solcher Operationen am offenen Herzen eingesetzt. Für Patienten, welche kurz- oder mittelfristig ein schwaches Herz oder eine schwache Lunge haben, kommen ECMO-Geräte (ECMO-Extracorporeal Membranoxygenator) als Unterstützung der schwachen Organe im Sinne eines außerhalb des Körpers des Patienten angeordneten Oxygenators zur Anwendung. Auch ein ECMO-Kreislauf besteht, wie die Herz-Lungen-Maschine, aus Blutpumpe, Oxygenator und Wärmetauscher, ist jedoch kompakter, einfacher und auch portabel.

Solche Operationen und andere notfall- und intensivmedizinischen Eingriffe sollen in Zukunft in Verbindung mit einer Magnetresonanztomographie des Patienten durchgeführt werden und die Informationen über die Ausdehnung und Lokalisierung ischämischer Bereiche, den Gefäßstatus und die Differenzierung einer möglichen Wiederbelebung des risikogefährdeten Gewebes liefern. Dabei kann eine bedingt MR-taugliche kardiopulmonale Unterstützung erforderlich sein, die Sicherheit garantiert und neben einem

*Magnetresonanztomographen* (MRT) ordnungsgemäß funktioniert. Zwar können Untersuchungen mit Herz-Lungen-Maschinen bereits heute in einem MRT durchgeführt werden. Dafür sind jedoch erhebliche Modifikationen und Sicherheitsvorkehrungen mit Auslagerung des gesamten Systems - bis auf das Schlauchsystem - notwendig. Jedoch ist gerade die Blutpumpe das Herzstück der Herz-Lungen-Maschine und diese sollte stets nah am Patienten betrieben werden können. Eine MR-taugliche Blutpumpe ist zurzeit nicht erhältlich.

Damit eine Blutpumpe in der Nähe zum MRT betrieben werden kann, müssen folgende Kriterien erfüllt werden:
- keine oder kaum ferromagnetische Komponenten, da diese vom MRT angezogen werden; angezogene Komponenten können leicht zu Geschossen werden und stellen somit ein Sicherheitsrisiko dar.
- keine oder kaum elektrisch leitende Komponenten, welche bewegt oder rotiert werden, da sonst Wirbelströme auftreten, welche auf die Komponenten als Drehmomente wirken oder erhitzen können.
- kein elektromagnetischer Antrieb.

Ein Gerät mit der Klassifizierung MR Conditional, sprich bedingt MR-taugliches oder bedingt MR-geeignetes Gerät darf gemäß den Standards ASTM F2503 während der Bildgebung keine Artefakte erzeugen und befindet sich außerhalb des Bildbereiches.

Da übliche extrakorporale Kreisläufe bislang nicht / bedingt MR-tauglich oder MR-sicher sind, und da die in den üblichen extrakorporalen Kreisläufen als Hauptflüssigkeitspumpe verwendeten Rollerpumpen und Axialpumpen allesamt nicht MR-tauglich waren, hatte man bislang den kardiopulmonalen Bypass für Anwendungen im MRT über sehr lange Blutschläuche oder mit einem Pumpenkopf bewerkstelligt, welcher über eine lange Faserverbundwelle mit einem weit vom MRT entfernt installierten Elektromotor angetrieben wurde. Für den klinischen Einsatz sind solche Lösungen untauglich.

Es besteht daher insbesondere die Aufgabe, eine extrakorporalen Kreislaufunterstützung der eingangs erwähnten Art zu schaffen, die kompakt ausgestaltet und auch bei kleinen Patienten, wie beispielsweise bei Neugeborenen und Säuglingen, zur MR-bedingten kardiopulmonalen Unterstützung verwendet werden kann.

Die erfindungsgemäße Lösung dieser Aufgabe besteht insbesondere darin, dass der Pumpenantrieb zumindest bedingt MR-tauglich ausgestaltet und als Gasexpansionsmotor ausgebildet ist. Hierbei ist eine MR-sichere Ausgestaltung mit umfasst.

Auch die erfindungsgemäße extrakorporale Kreislaufunterstützung weist eine Blut- oder Flüssigkeits-Hauptpumpe auf, deren Pumpeneinlass über wenigstens eine erste Flüssigkeitsleitung und deren Pumpenauslass über mindestens eine zweite Flüssigkeitsleitung an den Blutkreislauf eines Patienten anschließbar ist. Dabei ist der Blut- oder Flüssigkeits-Hauptpumpe der erfindungsgemäßen extrakorporalen Kreislaufunterstützung ein Pumpenantrieb zugeordnet, der die Flüssigkeits-Hauptpumpe antreibt. Erfindungsgemäß ist vorgesehen, dass auch dieser Pumpenantrieb bedingt MR-tauglich ausgestaltet und daher als Gasexpansionsmotor ausgebildet ist. Ein solcher Gasexpansionsmotor kann auch ohne störende ferromagnetische Komponenten zusammengebaut werden, die anderenfalls vom MRT angezogen würden. Darüber hinaus kommt der als Pumpenantrieb verwendete Gasexpansionsmotor ohne störende, elektrisch leitende Komponenten aus, welche bewegt oder rotiert werden, und die anderenfalls Wirbelströme erzeugen könnten. Insbesondere hat der als Pumpenantrieb verwendete Gasexpansionsmotor keinerlei elektromagnetischen Antrieb, sodass bei der erfindungsgemäßen extrakorporalen Kreislaufunterstützung über die Flüssigkeits-Hauptpumpe und den Oxygenator hinaus auch der Pumpenantrieb bedingt MR-tauglich ausgestaltet und im Umfeld eines MRT-Gerätes verwendet werden kann. Dabei weist auch die erfindungsgemäße extrakorporale Kreislaufunterstützung einen Oxygenator auf zum Anreichern des in der mindestens einen, zum Patienten führenden zweiten Flüssigkeitsleitung geführten Blutes mit Sauerstoff. Die erfindungsgemäße extrakorporale Kreislaufunterstützung ist als Herz-Lungen-Maschine oder als ECMO-Gerät einsetzbar.

Um den Volumenstrom des dem Gasexpansionsmotor als Antriebsmedium zugeführten Druckgases steuern und einregeln zu können, ist vorteilhaft, dass dieser Volumenstrom regulierbar ist mittels wenigstens einem Proportionalventil, insbesondere einem Piezo-Ventil.

Um die als Antriebskraft dienende Drehbewegung des Pumpenantriebs in eine Pumpbewegung der Blutpumpe umzusetzen, ist es vorteilhaft, wenn der Gasexpansionsmotor eine Antriebswelle hat, die mit der Flüssigkeits-Hauptpumpe in Antriebsverbindung steht.

Um bei einem Ausfall der Energieversorgung des Pumpenantriebs die Kreislaufunterstützung des Patienten sicherzustellen, ist es vorteilhaft, wenn am Antriebsstrang wenigstens ein Anschluss für eine Notkurbel vorgesehen ist. Ebenso kann eine vorzugsweise abnehmbare Notkurbel vorgesehen sein. Mittels der Notkurbel kann die Flüssigkeits-Hauptpumpe manuell angetrieben werden. Dies kann beispielsweise erforderlich sein, wenn während eines mobilen Betriebs der Kreislaufunterstützung unter Verwendung einer Gaskartusche zum Antrieb des Gasexpansionsmotors die Gaskartusche entleert wird.

Da der Gasexpansionsmotor nicht beliebig hohe und tiefe Drehzahlen und Drehmomente erzeugen kann, sieht eine bevorzugte Ausführungsform gemäß der Erfindung vor, dass im Antriebsstrang zwischen dem Gasexpansionsmotor und der Flüssigkeits-Hauptpumpe ein Getriebe vorgesehen ist. Dieses Getriebe überträgt das vom Antrieb erzeugte Drehmoment auf den Pumpenkopf.

Dabei kann es vorteilhaft sein, wenn das Getriebe als stufenloses Getriebe, als Zahnrad- oder als Planetengetriebe oder als hydrodynamischer Drehmomentwandler ausgestaltet ist.

Vorteilhaft kann es sein, wenn der zuvor erwähnte Anschluss für die Notkurbel und/oder die Notkurbel dabei im Antriebsstrang vor einem, beispielsweise vor dem zuvor erläuterten, Getriebe angeordnet ist, das zwischen dem Gasexpansionsmotor und der Flüssigkeits-Hauptpumpe zwischengeschaltet sein kann. So kann eine über die Notkurbel manuell eingeleitete Antriebsbewegung über das Getriebe eingeleitet und von diesem umgesetzt werden. Durch diese Getriebeunterstützung ist es gegebenenfalls leichter möglich, auch im manuellen Notbetrieb eine bestimmte Mindestdrehzahl der Flüssigkeits-Hauptpumpe zu erreichen, die zum ordnungsgemäßen Betrieb der Flüssigkeits-Hauptpumpe erforderlich sein kann.

Um weitere störende Einflüsse der im Umfeld eines MRT verwendeten erfindungsgemäßen extrakorporalen Kreislaufunterstützung möglichst zu vermeiden, ist es vorteilhaft, wenn die Antriebswelle und vorzugsweise alle im Antriebsstrang vorgesehenen Wellen aus vorzugsweise steifen Faserverbundwerkstoffen hergestellt sind.

Zu dem gleichen Zweck kann es sinnvoll sein, wenn die Antriebswelle und vorzugsweise alle im Antriebsstrang vorgesehenen Wellen in Keramik-Kugellagern und/oder in Kunststoffbuchsen gelagert sind.

Vorteilhaft kann es sein, wenn die Flüssigkeits-Hauptpumpe, die das Medium befördert, als Rollerpumpe, Peristaltikpumpe oder als Schlauchpumpe ausgebildet ist. Dabei befindet sich das Medium in einem als Flüssigkeitsleitung dienenden Schlauch des kardiopulmonalen Bypasses, wobei eine oder mehrere Rollen dieser Flüssigkeits-Hauptpumpe über den Schlauch walken und das im Schlauch befindliche Medium dadurch befördern.

Es können okklusive und nicht-okklusive Rollerpumpen eingesetzt werden, wobei bei okklusiven Rollerpumpen der verwendete Schlauch ganz zusammengedrückt wird, während nicht-okklusive Rollerpumpen den Schlauch entweder nicht komplett oder zumindest nicht gegen eine Außenkontur drücken.

Eine weitere vorteilhafte Ausführungsform gemäß der Erfindung sieht vor, dass die Flüssigkeits-Hauptpumpe als Strömungspumpe ausgebildet ist. Dabei kann diese Strömungspumpe als Kreiselpumpe oder als Axialpumpe ausgebildet sein. Möglich ist auch, dass die Flüssigkeits-Hauptpumpe als Impeller-Pumpe ausgebildet ist. Für die Anwendung der erfindungsgemäßen Maschine auch als ECMO-Maschine kann zusätzlich eine Ballonpulsation verwendet werden.

Um die sichere Verwendung der extrakorporalen Kreislaufunterstützung in Zusammenhang mit einer MRT-Untersuchung zu gewährleisten, ist es von Vorteil, wenn bewegliche Teile des Getriebes und/oder der Flüssigkeits-Hauptpumpe metallfrei ausgeführt sind. So kann die Verwendung ferromagnetischer Stoffe vermieden werden. Die beweglichen Teile können beispielsweise aus Kunststoff und/oder Keramik bestehen. Besonders vorteilhaft ist es, wenn die beweglichen Teile MR-sicher ausgeführt sind. Somit kann eine Kreislaufunterstützung realisiert werden, bei der weder im Getriebe noch in der Flüssigkeits-Hauptpumpe antriebsrelevante Metallteile verbaut sind.

Vorteilhaft ist es, wenn als Antriebsmedium für den Gasexpansionsmotor Druckluft oder Stickstoff verwendbar ist. Da in jedem Operationssaal und jedem MRT-Raum Druckluft vorhanden ist, bietet sich die Verwendung von Druckluft auch als Antriebsmedium für den Gasexpansionsmotor der erfindungsgemäßen extrakorporalen Kreislaufunterstützung an. Denkbar wäre auch, statt Druckluft Stickstoff als Druckgas einzusetzen.

Um die erfindungsgemäße extrakorporale Kreislaufunterstützung auch für Patiententransporte zwischen Operationssaal und MRT-Raum zu verwenden, aber auch für den Einsatz bei ECMO-Patienten, kann es vorteilhaft sein, wenn die als Antriebsmedium verwendete Druckluft mittels eines vorzugsweise tragbaren oder verfahrbaren Kompressors der extrakorporalen Kreislaufunterstützung erzeugbar ist.

Es wird eine weiterbildende Ausführungsform gemäß der Erfindung bevorzugt, bei welcher der Gasexpansionsmotor als ferritfreier Gasexpansionsmotor und insbesondere als ferritfreier Radialkolbenmotor ausgebildet ist.

Gemäß einer vorteilhaften Ausführungsform kann eine Steuereinheit zur Steuerung einer Pumpeinheit vorgesehen sein, wobei die Pumpeinheit zumindest den Gasexpansionsmotor und die Flüssigkeits-Hauptpumpe umfasst. Hierbei kann die Steuereinheit von der Pumpeinheit distanziert angeordnet sein. Die Pumpeinheit kann hierbei distanziert von der Steuereinheit betreibbar sein. So kann die Pumpeinheit nah am Patienten und somit in größerer Nähe zu einem MRT als die Steuereinheit betrieben werden. Einflüsse des MRT auf die Steuereinheit und umgekehrt können so vermindert oder vermieden werden. Es können auch die an die Steuereinheit in Bezug auf MR-Sicherheit gestellten Anforderungen reduziert werden.

Die Steuereinheit kann in englischer Sprache als Controlling Unit bezeichnet werden.

Hierbei kann die Steuereinheit über eine Druckluftleitung und/oder eine elektrische Verbindung mit der Pumpeinheit verbunden sein. Es ist vorteilhaft, wenn die elektrische Verbindung elektromagnetisch geschirmt ist.

Bei einer vorteilhaften Ausgestaltung kann ein Flusssensor vorgesehen sein, der zur Messung einer Flussgeschwindigkeit innerhalb einer Flüssigkeitsleitung der extrakorporalen Kreislaufunterstützung eingerichtet ist. So kann die Flussgeschwindigkeit des Blutes genau geregelt werden. Besonders vorteilhaft ist es, wenn es sich hierbei um einen nicht-invasiven Flusssensor handelt. Ein derartiger Sensor kann einerseits mehrfach verwendet werden, andererseits wird ein schädigender Einfluss des Flusssensors auf das Blut vermieden.

Vorteilhaft ist es, einen Drehzahlmesser vorzusehen, der zur zumindest mittelbaren Bestimmung einer Drehzahl der Flüssigkeits-Hauptpumpe eingerichtet ist. Dies kann beispielsweise eine Drehzahl einer zwischen Getriebe und Flüssigkeits-Hauptpumpe befindlichen Abtriebswelle sein. Vorzugsweise kann der Drehzahlmesser elektromagnetisch geschirmt und beispielsweise in einer Abschirmung aus Kupfer angeordnet sein. Eine elektrische Verbindung, vorzugsweise eine Signalverbindung, des Drehzahlmessers mit einer Steuereinheit ist vorzugsweise ebenfalls elektromagnetisch geschirmt ausgebildet. Eine Störung der Steuereinheit durch in eine elektrische Leitung eingespeiste Störsignale kann so vermieden werden.

Der Drehzahlmesser kann auch als Encoder ausgebildet sein. Somit ist es möglich, die Drehzahl einer Antriebs- bzw. Abtriebswelle zu erfassen und zur genauen Ansteuerung des Gasexpansionsmotors oder des Getriebes zu verwenden. Sind sowohl Flusssensor als auch Drehzahlmesser vorgesehen, können Flussgeschwindigkeit und Drehzahl gegenseitig als Kontrollwerte fungieren. Abweichungen von einem bekannten Verhältnis beider Werte zueinander können auf mögliche Störungen, beispielsweise durch den Betrieb des MRT, hinweisen.

Gemäß einer vorteilhaften Ausführungsform ist an einem Gehäuse der extrakorporalen Kreislaufunterstützung eine Markierung vorgesehen, die einen Rückschluss auf eine Ausrichtung einer dem Gasexpansionsmotor zugehörigen Antriebswelle ermöglicht.

Hierbei kann es sich um eine optisch wahrnehmbare Markierung wie etwa einen Pfeil handeln. Die Ausrichtung der Antriebswelle ist von außen zu erkennen und die Kreislaufunterstützung kann so in einem äußeren Magnetfeld ausgerichtet werden, dass ein störender Einfluss des Magnetfeldes, beispielsweise in Form eines Abbremsen und/oder einer Blockade des Antriebs, des Gasexpansionsmotors und/oder der Antriebswelle vermindert und/oder vermieden wird.

Weiter ist zur Lösung der eingangs genannten Aufgabe erfindungsgemäß bei einer MRT-Anordnung mit einem Magnetresonanztomographen (MRT) und einer erfindungsgemäßen extrakorporalen Kreislaufunterstützung vorgesehen, dass eine dem Gasexpansionsmotor zugehörige Antriebswelle parallel zu einer Längsmittelachse einer Ringanordnung von Spulen ausgerichtet ist. Alternativ oder zusätzlich kann vorgesehen sein, dass die genannte Antriebswelle parallel zu einer Längsmittelachse einer Untersuchungsöffnung des MRT ausgerichtet ist.

Somit kann ein störender Einfluss des MRT auf den Antrieb der Kreislaufunterstützung verringert oder vermieden werden. Bei einer anderen Ausrichtung der Antriebswelle bzw. des Gasexpansionsmotors kann sich ein störender Einfluss des MRT in Form eines Abbremsen des Antriebs manifestieren. Die Untersuchungsöffnung ist in der Regel zylindrisch ausgeführt und wird durch die Öffnung der Ringanordnung von Spulen definiert. Die Spulen umfassen beispielsweise Spulen zur Erzeugung des Magnetfeldes und Hochfrequenzspulen. Die Einstellung der korrekten Ausrichtung der Antriebswelle kann insbesondere durch die bereits beschriebene Markierung an einem Gehäuse der Kreislaufunterstützung unterstützt werden.

Weiterbildungen gemäß der Erfindung ergeben sich aus der nachfolgendenden Beschreibung eines erfindungsgemäßen Ausführungsbeispiels in Verbindung mit den Ansprüchen sowie der Zeichnung. Nachstehend wird die Erfindung anhand eines bevorzugten Ausführungsbeispiels noch näher beschrieben.

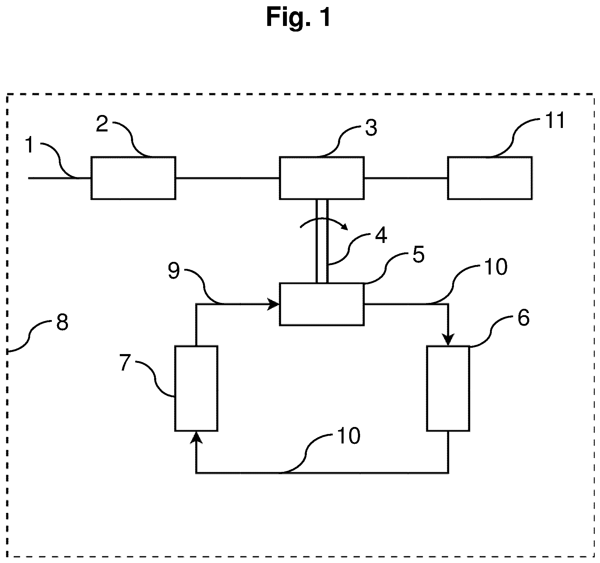

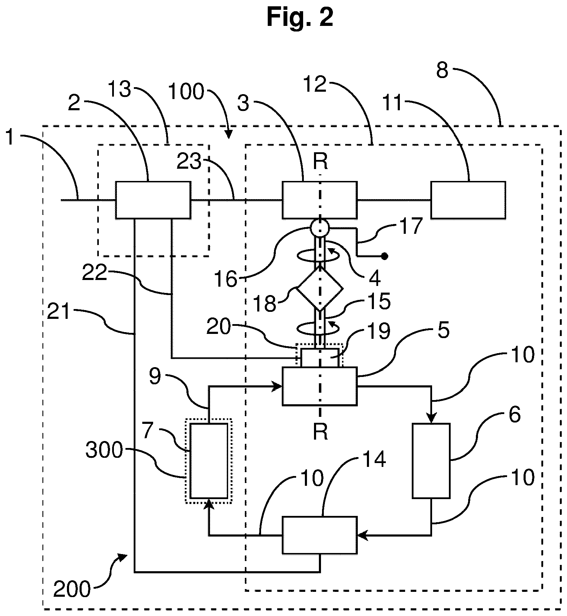

In Figur 1 ist eine extrakorporale Kreislaufunterstützung in einer Schemazeichnung dargestellt, die in unmittelbarer Nähe zu einem MRT und somit zu einem Patienten eingesetzt werden kann. In Figur 2 ist eine weitere schematisierte Darstellung der extrakorporalen Kreislaufunterstützung mit weiteren Komponenten gezeigt.

Die extrakorporale Kreislaufunterstützung ist als eine Vorrichtung zur extrakorporalen Kreislaufunterstützung eines Patienten 7 ausgebildet und in Figur 2 im Ganzen mit 100 bezeichnet. Figur 2 zeigt außerdem eine erfindungsgemäße MRT-Anordnung 200, die eine extrakorporale Kreislaufunterstützung 100 sowie ein MRT 300 umfasst.

In dem hier gezeigten Ausführungsbeispiel ist der extrakorporale Kreislauf in einem MRT-Raum 8 eingesetzt. Die hier gezeigte extrakorporale Kreislaufunterstützung 100 weist eine Blut- oder Flüssigkeits-Hauptpumpe 5 auf, deren Pumpeneinlass über wenigstens eine erste Flüssigkeitsleitung 9 und deren Pumpenauslass über mindestens eine zweite Flüssigkeitsleitung 10 an den Blutkreislauf eines Patienten 7 anschließbar ist. Die hier gezeigte extrakorporale Kreislaufunterstützung 100 hat einen Oxygenator 6, der zum Anreichern des in der mindestens einen zweiten Flüssigkeitsleitung 9, 10 zum Patienten 7 hin geführten Blutes mit Sauerstoff sowie zur Elimination des im Blut befindlichen Kohlendioxids bestimmt ist. Um über die Flüssigkeits-Hauptpumpe 5 und den Oxygenator 6 hinaus auch den der Flüssigkeits-Hauptpumpe 5 zugeordneten Pumpenantrieb bedingt MR-tauglich auszugestalten, ist dieser Pumpenantrieb hier als Gasexpansionsmotor 3 ausgebildet. Dieser Gasexpansionsmotor 3 hat eine Antriebswelle 4, die mit der Blut- oder Flüssigkeits-Hauptpumpe 5 in Antriebsverbindung steht. Diese Antriebswelle 4 und alle weiteren, im Antriebsstrang zwischen Pumpenantrieb und Flüssigkeits-Hauptpumpe 5 befindlichen Wellen sind aus vorzugsweise steifen Faserverbundwerkstoffen, wie zum Beispiel Carbon oder Glasfaserverbunde, hergestellt und in Keramikkugellagern und/oder Kunststoffbuchsen gelagert. Über eine Zufuhrleitung 1 wird dem Gasexpansionsmotor 3 das als Antriebsmedium dienende Druckgas zugeführt. Dabei ist in der Zufuhrleitung 1 ein Regelventil 2 zwischengeschaltet, mit dem der Volumenstrom des dem Gasexpansionsmotor 3 als Antriebsmedium zugeführten Druckgases regulierbar ist. Dieses Regelventil 2 ist vorzugsweise als Piezo-Ventil ausgestaltet.

Um die Drehzahl der vom Gasexpansionsmotor 3 angetriebenen Welle 4 in einem breiten Drehzahlbereich einregeln und das Drehmoment des Gasexpansionsmotors 3 variieren zu können, kann im Antriebsstrang zwischen dem Gasexpansionsmotor 3 und der Flüssigkeits-Hauptpumpe 5 ein Getriebe vorgesehen sein. Das hier nicht weiter dargestellte Getriebe kann als stufenloses Getriebe (CVT-Continuous-Variable-Transmission), beispielsweise als Keilriemenvariator oder als NuVinci-Getriebe ausgestaltet sein. Möglich ist auch, dass das Getriebe als Zahnradgetriebe, Planetengetriebe oder als hydrodynamischer Drehmomentwandler ausgebildet ist.

Die Flüssigkeits-Hauptpumpe 5 kann als Strömungspumpe, beispielweise als Kreiselpumpe oder als Axialpumpe, ausgebildet sein. Möglich ist auch, dass die Flüssigkeits-Hauptpumpe 5 als Impeller-Pumpe ausgebildet ist. In dem hier gezeigten Ausführungsbeispiel ist die Flüssigkeits-Hauptpumpe 5 als Roller- oder Peristaltik-Pumpe ausgebildet. Dabei befindet sich das Fördermedium in einem Schlauch, über den eine oder mehrere Rollen walken. Die im Wesentlichen aus der als Pumpenkopf ausgebildeten Flüssigkeits-Hauptpumpe 5, dem als Pumpenantrieb dienenden Gasexpansionsmotor 3, dem Oxygenator 6 und dem, aus den Flüssigkeitsleitungen 9 und 10 gebildeten kardiopulmonalen Bypass bestehende Herz-Lungen-Maschine steht zur Parametereinstellung und -überwachung mit einer Steuereinheit 13 in Steuerverbindung, die beispielsweise als vorzugsweise tragbare Datenverarbeitungsanlage oder als Notebook ausgebildet sein kann. Dank der erfindungsgemäßen Ausgestaltung der wesentlichen Komponenten der hier schematisch dargestellten Herz-Lungen-Maschine kann diese in einem MRT-Raum innerhalb der MRT-Umgebung platziert werden. Im gezeigten Ausführungsbeispiel umfasst die Steuereinheit das Regelventil 2, sie kann jedoch auch separat ausgeführt sein.

Die mit Hilfe des Gasexpansionsmotors 3 drehangetriebene Haupt-Flüssigkeitspumpe 5 ist hier als Peristaltik-Pumpe ausgebildet, die mehrere Rollen hat, welche im Pumpenkopf auf dem das Fördermedium führenden Schlauch walken. Das Quetschen des Schlauches und die Korrektur der Okklusions-Einstellung sind von grundlegender Bedeutung, um einen Rückfluss mit einer Erhöhung der kinetischen Energie (nicht okklusiv oder unterokklusiv), eine Abnahme der Lebensdauer der Schläuche durch Spallation (Über-Okklusion) oder Hämolyse zu vermeiden. Dabei hat sich eine "just-occlusive"-Einstellung ohne retrograde Strömung und minimierte Spallation als optimale Einstellung gezeigt. Das Blut des Patienten 6 wird der Blut- oder Haupt-Flüssigkeitspumpe 5 über eine erste Flüssigkeitsleitung 9 zugeführt, wobei die Blut- oder Haupt-Flüssigkeitspumpe 5 das Blut anschließend über eine zweite Flüssigkeitsleitung 10, in die der Oxygenator 6 sowie ggf. auch ein Wärmetauscher zwischengeschaltet ist, gefördert. Um die Durchblutungsrate des Patienten genau zu messen, kann eine Ultraschall-Durchflusssonde eingesetzt werden, während der Druck mit Hilfe eines Druckwandlers gemessen und an die Steuereinheit weitergegeben wird. Der verwendete Oxygenator 6, die Kanülen, als Flüssigkeitsleitungen dienende Schläuche sowie die Blutpumpe sind bedingt MR-tauglich oder MR-sicher und können so nah wie möglich am Patienten platziert werden, um das Füllungsvolumen des extrakorporalen Kreislaufs zu minimieren.

Über die Zufuhrleitung 1 wird dem Gasexpansionsmotor 3 das erforderliche Druckgas, hier Druckluft, zugeführt. Das in die Zufuhrleitung 1 zwischengeschaltete Regelventil 2 regelt den Luftstrom, der den Gasexpansionsmotor 3 antreibt. Die vom Gasexpansionsmotor 3 abströmende Luft wird vom Patienten weggeleitet und durch einen Schalldämpfer 11 diffundiert. Während der Gasexpansionsmotor 3 und der Schalldämpfer 11 neben dem MRT platziert werden können, befindet sich das hier als Piezo-Ventil ausgebildete Druckregelventil 2 außerhalb der 20-mT-Linie, während demgegenüber der Gasexpansionsmotor 3, der der Gasexpansionspumpe 3 zugeordnete Geräuschdämpfer 11, die Antriebswelle 4, die Flüssigkeits-Hauptpumpe 5 sowie der Oxygenator 6 mitsamt dem Patienten 7 sich innerhalb der 20-mT-Linie des MRT-Raums 8 befinden. Die beispielsweise durch ein Notebook gebildete Steuereinheit und die dazugehörigen Anzeigen können sich in einem abgeschirmten Gehäuse, welches die Emission hochfrequenter elektromagnetischer Strahlung in die MRT-Umgebung verhindert, befinden.

Die verschiedenen Elemente der gezeigten extrakorporalen Kreislaufunterstützung 100 lassen sich in eine Pumpeinheit 12 und eine Steuereinheit 13 zusammenfassen. Die Steuereinheit 13 ist von der Pumpeinheit 12 distanziert ausgebildet und mit dieser über eine Fluidleitung, hier eine Druckluftleitung 23, und elektromagnetisch geschirmte elektrische Verbindungen 21, 22 verbunden. Die Steuereinheit 13 umfasst ferner einen nicht-invasiven Flusssensor 14, mit dem die Flussgeschwindigkeit des Blutes bestimmt wird.

Am Antriebsstrang der extrakorporalen Kreislaufunterstützung 100, genauer an der Antriebswelle 4 zwischen Gasexpansionsmotor 3 und Getriebe 18 ist ein Anschluss 16 für eine abnehmbare Notkurbel 17 vorgesehen. Die beweglichen Teile des Getriebes 18 und der Flüssigkeits-Hauptpumpe 5 sind metallfrei und MR-sicher ausgeführt, wobei sie Teile aus Kunststoff und Keramik umfassen.

Der Anschluss 16 und somit auch die Notkurbel 17 sind hierbei im Antriebsstrang vor dem Getriebe 18 angeordnet. So kann eine über die Notkurbel 17 manuell eingeleitete Antriebsbewegung über das Getriebe 18 eingeleitet und von diesem umgesetzt werden. Durch diese Getriebeunterstützung ist es leichter möglich, auch im manuellen Notbetrieb eine bestimmte Mindestdrehzahl der Flüssigkeits-Hauptpumpe 5 zu erreichen, die zum ordnungsgemäßen Betrieb der Flüssigkeits-Hauptpumpe 5 erforderlich ist.

Ferner ist ein Drehzahlmesser 19 ausgebildet, welcher eine Drehzahl einer zwischen Getriebe 18 und Flüssigkeits-Hauptpumpte 5 befindlichen Abtriebswelle 15 bestimmt. Der Drehzahlmesser 19 ist als Encoder ausgebildet und von einer elektromagnetischen Abschirmung 20 umgeben.

Der Patient 7 ist von einem MRT 300 umgeben. Extrakorporale Kreislaufunterstützung 100 und MRT 300 bilden eine erfindungsgemäße MRT-Anordnung 200, bei der eine Rotationsachse R der Antriebswelle 4 des Gasexpansionsmotors 3 parallel zu einer Längsmittelachse einer Ringanordnung von Spulen und einer Untersuchungsöffnung des MRT 300 ausgerichtet ist.

MR-sichere Gegenstände sind als Gegenstände definiert, die vollständig aus elektrisch nicht-leitenden, nicht-metallischen und nicht-magnetischen Materialien bestehen und somit keine Gefahren darstellen und zudem keine Bildartefakte erzeugen, während Gegenstände, die als bedingt MR-tauglich gekennzeichnet sind, in MRT-Umgebungen unter definierten Bedingungen erlaubt sind, und keine Bildstörungen verursachen. MR-unsichere Gegenstände stellen ein inakzeptables Risiko für den Patienten dar und sind daher verboten. Die meisten MRT-Räume haben eine gezeichnete Linie auf dem Boden, welche die 20-mT-Linie anzeigt. Bedingt MR-taugliche elektronische Geräte, wie beispielsweise MR-Anästhesie-Monitore oder MRmedizinische Beatmungsgeräte, sollten außerhalb dieser 20-mT-Linie bleiben. Nach den internationalen MRT-Sicherheitsstandards darf ein bedingt MR-taugliches Gerät während der Bildgebung keine Artefakte erstellen. Die den Gasexpansionsmotor 3 und die Flüssigkeits-Hauptpumpe 5 aufweisende Pumpeneinheit der hier dargestellten extrakorporalen Kreislaufunterstützung 100 wird komplett aus MR-sicheren und bedingt MR-tauglichen Materialien hergestellt. Daher sind keine Tests auf Verdrängungskräfte oder Magnetfeldinduziertes Drehmoment erforderlich und auch die Prüfung von Bildartefakten kann unterbleiben, da keines der Geräte unmittelbar im oder unmittelbar angrenzend am Bildbereich des MRTs platziert wird. Abgesehen von der hier nicht weiter dargestellten Steuereinheit können im Wesentlichen alle hier dargestellten Komponenten in ca. 1 m Entfernung vom ISO-Zentrum des MRT aufgestellt werden. Lediglich das als Regelventil dienende Piezo-Ventil 2 sowie ein spezieller Faraday'scher Käfig mit Steuerung und Datenaufzeichnung sind etwa 3 m weit weg vom ISO-Zentrum des MRT zu platzieren. Diese Distanzangaben sind in Verbindung mit einem 3-Tesla-MRT empfohlen und sollten auch für ein 1-Tesla- und 1.5-Tesla-MRT gelten.

### Bezugszeichenliste

- 1: Druckgas- oder Druckluft-Zufuhrleitung
- 2: Regelventil, insbesondere Piezo-Ventil
- 3: Gasexpansionsmotor
- 4: Antriebswelle
- 5: Blut- oder Flüssigkeits-Hauptpumpe
- 6: Oxygenator
- 7: Patient
- 8: MRT-Raum
- 9: erste Flüssigkeitsleitung
- 10: zweite Flüssigkeitsleitung
- 11: Geräusch- oder Schalldämpfer
- 12: Pumpeinheit
- 13: Steuereinheit
- 14: Flusssensor
- 15: Abtriebswelle
- 16: Anschluss
- 17: Notkurbel
- 18: Getriebe
- 19: Drehzahlmesser
- 20: Abschirmung21 Verbindung
- 22: Verbindung
- 23: Druckluftleitung
- 100: extrakorporale Kreislaufunterstützung
- 200: MRT-Anordnung
- 300: MRT
- R: Rotationsachse

## Patentansprüche

1. Vorrichtung zur extrakorporalen Kreislaufunterstützung (100) mit einer Flüssigkeits-Hauptpumpe (5), deren Pumpeneinlass über wenigstens eine erste Flüssigkeitsleitung (9) und deren Pumpenauslass über mindestens eine zweite Flüssigkeitsleitung (10) an den Blutkreislauf eines Patienten (7) anschließbar ist, mit einem Oxygenator (6) zum Anreichern des in der mindestens einen zweiten Flüssigkeitsleitung (10) geführten Blutes mit Sauerstoff, sowie mit einem Pumpenantrieb, der die Flüssigkeits-Hauptpumpe (5) antreibt und als Gasexpansionsmotor (3) ausgebildet ist, **dadurch gekennzeichnet, dass** der Pumpenantrieb MR-conditional, sprich bedingt MR-tauglich ausgestaltet ist.

2. Vorrichtung zur extrakorporalen Kreislaufunterstützung (100) nach Anspruch 1, **dadurch gekennzeichnet, dass** der Volumenstrom des dem Gasexpansionsmotor (3) als Antriebsmedium zugeführten Druckgases mittels wenigstens einem Piezo-Ventil (2) regulierbar ist.

3. Vorrichtung zur extrakorporalen Kreislaufunterstützung (100) nach Anspruch 1 oder 2, **dadurch gekennzeichnet, dass** der Gasexpansionsmotor (3) eine Antriebswelle (4) hat, die mit der Flüssigkeits-Hauptpumpe (5) in Antriebsverbindung steht.

4. Vorrichtung zur extrakorporalen Kreislaufunterstützung (100) nach einem der Ansprüche 1 bis 3, **dadurch gekennzeichnet, dass** im Antriebsstrang zwischen dem Gasexpansionsmotor (3) und der Flüssigkeits-Hauptpumpe (5) ein Getriebe (18) vorgesehen ist.

5. Vorrichtung zur extrakorporalen Kreislaufunterstützung (100) nach Anspruch 4, **dadurch gekennzeichnet, dass** das Getriebe (18) als stufenloses Getriebe (18), als Zahnrad- oder Planetengetriebe oder als hydrodynamischer Drehmomentwandler ausgestaltet ist.

6. Vorrichtung zur extrakorporalen Kreislaufunterstützung (100) nach einem der Ansprüche 1 bis 5, **dadurch gekennzeichnet, dass** die Antriebswelle (4) des Gasexpansionsmotors (3) und vorzugsweise alle im Antriebsstrang vorgesehenen Wellen (4, 15) aus vorzugsweise steifen Faserverbundwerkstoffen hergestellt sind und/oder in Keramikkugellagern und/oder in Kunststoffbuchsen gelagert sind.

7. Vorrichtung zur extrakorporalen Kreislaufunterstützung (100) nach einem der Ansprüche 1 bis 6, **dadurch gekennzeichnet, dass** die Flüssigkeits-Hauptpumpe (5) als Rollerpumpe, als Peristaltikpumpe, als Schlauchpumpe, als Strömungspumpe, als Kreiselpumpe, als Axialpumpe oder als Impellerpumpe ausgebildet ist.

8. Vorrichtung zur extrakorporalen Kreislaufunterstützung (100) nach einem der Ansprüche 1 bis 7, **dadurch gekennzeichnet, dass** bewegliche Teile des Getriebes (18) und/oder der Flüssigkeits-Hauptpumpe (5) metallfrei, insbesondere MR-sicher ausgeführt sind und/oder aus Kunststoff und/oder aus Keramik bestehen.

9. Vorrichtung zur extrakorporalen Kreislaufunterstützung (100) nach einem der Ansprüche 1 bis 8, **dadurch gekennzeichnet, dass** der Gasexpansionsmotor (3) als Lamellen-, Turbinen-, Zahnrad- oder als Axial- oder Radialkolbenmotor und/oder als ferritfreier Gasexpansionsmotor (3) ausgebildet ist.

10. Vorrichtung zur extrakorporalen Kreislaufunterstützung (100) nach einem der Ansprüche 1 bis 9, mit einer Steuereinheit (13) zur Steuerung einer zumindest den Gasexpansionsmotor (3) und die Flüssigkeits-Hauptpumpe (5) umfassenden Pumpeinheit (12), vorzugsweise wobei die Steuereinheit (13) von der Pumpeinheit (12) distanziert angeordnet und/oder die Pumpeinheit (12) distanziert von der Steuereinheit (12) betreibbar ist.

11. Vorrichtung zur extrakorporalen Kreislaufunterstützung (100) nach Anspruch 10, **dadurch gekennzeichnet, dass** die Steuereinheit (13) über eine Druckluftleitung (23) und/oder eine insbesondere elektromagnetisch geschirmte elektrische Verbindung (21, 22) mit der Pumpeinheit (12) verbunden ist.

12. Vorrichtung zur extrakorporalen Kreislaufunterstützung (100) nach einem der Ansprüche 1 bis 11, mit einem vorzugsweise nicht-invasiven Flusssensor (14), der zur Messung einer Flussgeschwindigkeit innerhalb einer Flüssigkeitsleitung (9,10) der extrakorporalen Kreislaufunterstützung (100) eingerichtet ist.

13. Vorrichtung zur extrakorporalen Kreislaufunterstützung (100) nach einem der Ansprüche 1 bis 12, mit einem Drehzahlmesser (19), der zur zumindest mittelbaren Bestimmung einer Drehzahl der Flüssigkeits-Hauptpumpe eingerichtet ist, vorzugsweise wobei der Drehzahlmesser (19) als Encoder ausgebildet und/oder elektromagnetisch geschirmt ist.

14. Vorrichtung zur extrakorporalen Kreislaufunterstützung (100) nach einem der Ansprüche 1 bis 13, mit einer Markierung, die einen Rückschluss auf eine Ausrichtung einer Antriebswelle (4) des Gasexpansionsmotors (4) ermöglicht, vorzugsweise wobei die Markierung an einem Gehäuse der extrakorporalen Kreislaufunterstützung (100) angeordnet ist.

15. Vorrichtung zur extrakorporalen Kreislaufunterstützung (100) nach einem der Ansprüche 1 bis 14, **dadurch gekennzeichnet, dass** die extrakorporale Kreislaufunterstützung (100) als Herz-Lungen-Maschine oder als extrakorporale Membranoxygenierung (ECMO) ausgebildet ist.

16. MRT-Anordnung (200) mit einem Magnetresonanztomographen (MRT) (300) und einer Vorrichtung zur extrakorporalen Kreislaufunterstützung (100) nach einem der vorangehenden Ansprüche, **dadurch gekennzeichnet, dass** eine Rotationsachse (R) einer Antriebswelle (4) des Gasexpansionsmotors (3) parallel zu einer Längsmittelachse einer Ringanordnung von Spulen und/oder einer Untersuchungsöffnung des MRT (300) ausgerichtet ist.

## Claims

1. Device for extracorporeal circuit support (100) having a liquid primary pump (5), the pump inlet of which can be connected via at least one first liquid line (9) and the pump outlet of which can be connected via at least one second liquid line (10) to the circulatory system of a patient (7), having an oxygenator (6) for enriching with oxygen the blood conveyed in the at least one second liquid line (10), and having a pump drive which drives the liquid primary pump (5) and is configured as a gas expansion motor, **characterized in that** the pump drive is designed MR-conditionally, i.e. in a conditionally MR-compatible manner.

2. Device for extracorporeal circuit support (100) according to claim 1, **characterized in that** the volume flow rate of the compressed gas supplied to the gas expansion motor (3) as the working medium can be regulated by means of at least one piezo valve (2).

3. Device for extracorporeal circuit support (100) according to claim 1 or 2, **characterized in that** the gas expansion motor (3) has a drive shaft (4), which is operatively connected to the liquid primary pump (5).

4. Device for extracorporeal circuit support (100) according to one of claims 1 to 3, **characterized in that** a transmission (18) is provided in the drive train between the gas expansion motor (3) and the liquid primary pump (5).

5. Device for extracorporeal circuit support (100) according to claim 4, **characterized in that** the transmission (18) is designed as a continuously variable transmission (18), as a gear transmission or planetary transmission, or as a hydrodynamic torque converter.

6. Device for extracorporeal circuit support (100) according to one of claims 1 to 5, **characterized in that** the drive shaft (4) of the gas expansion motor (3) and preferably all shafts (4, 15) provided in the drive train are produced from preferably stiff fiber-reinforced composites and/or are mounted in ceramic ball bearings and/or in plastic bushes.

7. Device for extracorporeal circuit support (100) according to one of claims 1 to 6, **characterized in that** the liquid primary pump (5) is configured as a roller pump, peristaltic pump, flexible-tube pump, dynamic pump, rotary pump, axial-flow pump or impeller pump.

8. Device for extracorporeal circuit support (100) according to one of claims 1 to 7, **characterized in that** moving parts of the transmission (18) and/or of the liquid primary pump (5) are designed to be metal-free, in particular MR-safe, and/or consist of plastic and/or of ceramic.

9. Device for extracorporeal circuit support (100) according to one of claims 1 to 8, **characterized in that** the gas expansion motor (3) is configured as a multi-disk, turbine, gear, or axial or radial piston motor, or as a ferrite-free gas expansion motor (3).

10. Device for extracorporeal circuit support (100) according to one of claims 1 to 9, having a control unit (13) for controlling a pump unit (12) comprising at least the gas expansion motor (3) and the liquid primary pump (5), preferably wherein the control unit (13) is arranged at a distance from the pump unit (12) and/or the pump unit (12) can be operated at a distance from the control unit (12).

11. Device for extracorporeal circuit support (100) according to claim 10, **characterized in that** the control unit (13) is connected to the pump unit (12) via a compressed-air line (23) and/or an in particular electromagnetically shielded electrical connection (21, 22).

12. Device for extracorporeal circuit support (100) according to one of claims 1 to 11, having a preferably non-invasive flow sensor (14) for measuring a flow rate inside a liquid line (9, 10) of the extracorporeal circuit support (100).

13. Device for extracorporeal circuit support (100) according to one of claims 1 to 12, having a rotational-speed sensor (19) which is adapted for at least indirect determination of a rotational speed of the liquid primary pump, preferably wherein the rotational-speed sensor (19) is configured as an encoder and/or is electromagnetically shielded.

14. Device for extracorporeal circuit support (100) according to one of claims 1 to 13, having a marking which allows deduction of an alignment of a drive shaft (4) of the gas expansion motor (3), preferably wherein the marking is arranged on a housing of the extracorporeal circuit support (100).

15. Device for extracorporeal circuit support (100) according to one of claims 1 to 14, **characterized in that** the extracorporeal circuit support (100) is configured as a heart-lung machine or as extracorporeal membrane oxygenation (ECMO).

16. MRT arrangement (200) having a magnetic resonance tomograph (MRT) (300) and an extracorporeal circuit support (100) according to one of the preceding claims, **characterized in that** a rotation axis (R) of a drive shaft (4) of the gas expansion motor (3) is aligned parallel to a longitudinal midaxis of an annular arrangement of coils and/or an examination opening of the MRT (300).

## Revendications

1. Dispositif d'assistance circulatoire extracorporelle (100) avec une pompe principale à liquide (5), dont l'entrée de la pompe est raccordable au circuit sanguin d'un patient par l'intermédiaire d'au moins une première conduite de liquide (9) et dont la sortie de la pompe est raccordable au circuit sanguin d'un patient (7) par l'intermédiaire d'au moins une deuxième conduite de liquide (10), avec un oxygénateur (6) pour enrichir en oxygène le sang guidé dans l'au moins une deuxième conduite de liquide (10), ainsi qu'avec un entraînement de pompe qui entraîne la pompe principale à liquide (5) et qui est conçu comme un moteur à expansion de gaz (3), **caractérisée en ce que** l'entraînement de pompe est conçu de façon à être conditionnellement compatible avec la RM, c'est-à-dire compatible avec la RM sous certaines conditions.

2. Dispositif d'assistance circulatoire extracorporelle (100) selon la revendication 1, **caractérisé en ce que** le débit volumique du gaz comprimé amené au moteur à expansion de gaz (3) en tant que milieu d'entraînement est réglable au moyen d'au moins une soupape piézoélectrique (2).

3. Dispositif d'assistance circulatoire extracorporelle (100) selon la revendication 1 ou 2, **caractérisé en ce que** le moteur à expansion de gaz (3) a un arbre d'entraînement (4) qui est en liaison d'entraînement avec la pompe principale à liquide (5).

4. Dispositif d'assistance circulatoire extracorporelle (100) selon l'une des revendications 1 à 3, **caractérisé en ce qu'**une transmission (18) est prévue dans le groupe motopropulseur entre le moteur à expansion de gaz (3) et la pompe principale à liquide (5).

5. Dispositif d'assistance circulatoire extracorporelle (100) selon la revendication 4, **caractérisé en ce que** la transmission (18) est conçue comme une transmission à variation continue (18), comme un engrenage ou un engrenage planétaire ou comme un convertisseur de couple hydrodynamique.

6. Dispositif d'assistance circulatoire extracorporelle (100) selon l'une des revendications 1 à 5, **caractérisé en ce que** l'arbre d'entraînement (4) du moteur à expansion de gaz (3) et de préférence tous les arbres (4, 15) prévus dans le groupe motopropulseur sont fabriqués en matériaux composites fibreux de préférence rigides et/ou sont montés dans des roulements à billes en céramique et/ou dans des douilles en matière plastique.

7. Dispositif d'assistance circulatoire extracorporelle (100) selon l'une des revendications 1 à 6, **caractérisé en ce que** la pompe principale à liquide (5) est conçue comme une pompe à rouleaux, une pompe péristaltique, une pompe à tuyaux, une pompe de brassage, une pompe centrifuge, une pompe axiale ou une pompe à impulseur.

8. Dispositif d'assistance circulatoire extracorporelle (100) selon l'une des revendications 1 à 7, **caractérisé en ce que** les pièces mobiles de la transmission (18) et/ou de la pompe principale à liquide (5) sont réalisées sans métal, en particulier sans risque de RM, et/ou sont en matière plastique et/ou en céramique.

9. Dispositif d'assistance circulatoire extracorporelle (100) selon l'une des revendications 1 à 8, **caractérisé en ce que** le moteur à expansion de gaz (3) est conçu comme un moteur à lamelles, à turbine, à engrenages ou comme un moteur à pistons axiaux ou radiaux et/ou comme un moteur à expansion de gaz (3) sans ferrite.

10. Dispositif d'assistance circulatoire extracorporelle (100) selon l'une des revendications 1 à 9, avec une unité de commande (13) pour la commande d'une unité de pompage (12) comprenant au moins le moteur à expansion de gaz (3) et la pompe principale à liquide (5), l'unité de commande (13) étant disposée de préférence à distance de l'unité de pompage (12) et/ou l'unité de pompage (12) étant actionnable à distance de l'unité de commande (13).

11. Dispositif d'assistance circulatoire extracorporelle (100) selon la revendication 10, **caractérisé en ce que** l'unité de commande (13) est reliée à l'unité de pompage (12) par l'intermédiaire d'une conduite d'air comprimé (23) et/ou une liaison électrique (21, 22), en particulier à blindage électromagnétique.

12. Dispositif d'assistance circulatoire extracorporelle (100) selon l'une des revendications 1 à 11, comprenant un capteur de flux (14), de préférence non invasif, agencé pour mesurer une vitesse de flux à l'intérieur d'une conduite de liquide (9, 10) de l'assistance circulatoire extracorporelle (100).

13. Dispositif d'assistance circulatoire extracorporelle (100) selon l'une des revendications 1 à 12, comprenant un tachymètre (19) qui est agencé pour déterminer au moins indirectement une vitesse de rotation de la pompe principale à liquide, le tachymètre (19) étant conçu de préférence comme un encodeur et/ou blindé électromagnétiquement.

14. Dispositif d'assistance circulatoire extracorporelle (100) selon l'une des revendications 1 à 13, avec un repère qui permet de déduire une orientation d'un arbre d'entraînement (4) du moteur à expansion de gaz (4), le repère étant disposé de préférence sur un boîtier de l'assistance circulatoire extracorporelle (100).

15. Dispositif d'assistance circulatoire extracorporelle (100) selon l'une des revendications 1 à 14, **caractérisé en ce que** l'assistance circulatoire extracorporelle (100) est conçue comme une machine cœur-poumons ou comme une oxygénation par membrane extracorporelle (ECMO).

16. Agencement d'IRM (200) avec un tomographe à résonance magnétique (IRM) (300) et un dispositif d'assistance circulatoire extracorporelle (100) selon l'une des revendications précédentes, **caractérisé en ce qu'**un axe de rotation (R) d'un arbre d'entraînement (4) du moteur à expansion de gaz (3) est orienté parallèlement à un axe médian longitudinal d'un agencement annulaire de bobines et/ou d'une ouverture d'examen de l'IRM (300).
